# EUROPEAN PATENT APPLICATION

(11) **EP 1 403 384 A1**
(43) Date of publication of application: **31.03.2004**
(21) Application number: 02078992.1
(22) Date of filing: 26.09.2002
(51) Int. Cl.: C12Q 1/70

(54) **Method for detecting and typing of cutaneous HPV and primers and probes for use therein**

(71) Applicant: Stichting Researchfonds Pathologie, 1078 LW Amsterdam (NL)
(72) Inventor: Meijer, Christophorus Joannes Lambertus Maria, 2317 GV Leiden (NL); Snijders, Petrus Josephus Ferdinandus, 1187 VE Amstelveen (NL)
(74) Representative: Prins, Adrianus Willem

(57) **Abstract**

The present invention relates to a method for detecting and typing of human papillomavirus (HPV). In particular, the invention relates to a method for detecting and typing cutaneous HPVs of supergroup B and to primers and probes for use in a method of the invention. An important advantage of the present invention is that a large number of HPV types can be detected in a single assay, that it is applicable to fixed and poorly preserved material and that the detection method and typing method can be coupled.

SEQUENCE LISTING

<110>  Stichting Researchfonds Pathologie

<120>  Method for detecting and typing of cutaneous HPV and primers and probes
for use therein

<130>  P60890EP00

<140>  EP 02078992.1
<141>  2002-09-26

<160>  109

<170>  PatentIn version 3.1

<210>  1
<211>  21
<212>  DNA
<213>  Artificial Sequence

<220>
<223>  primer 5f

<400>  1
ttagctcaaa ttaatgcaat g                                                21


<210>  2
<211>  21
<212>  DNA
<213>  Artificial Sequence

<220>
<223>  primer 6f

<400>  2
ttggcacaaa ttaatgcaat g                                                21


<210>  3
<211>  21
<212>  DNA
<213>  Artificial Sequence

<220>
<223>  primer 7f

<400>  3
ttagcacaaa ttaatgctat g                                                21


<210>  4
<211>  21
<212>  DNA
<213>  Artificial Sequence

<220>
<223>  primer 8f

<400>  4

ttagctcaga taaatgcaat g                                                21

<210>  5
<211>  21
<212>  DNA
<213>  Artificial Sequence

<220>
<223>  primer 9f

<400>  5
ctggctcatt taaatgtaat g                                                21

<210>  6
<211>  28
<212>  DNA
<213>  Artificial Sequence

<220>
<223>  primer 5r-bio

<400>  6
atcaggggtg ggtacaaatc ctaactgc                                         28

<210>  7
<211>  28
<212>  DNA
<213>  Artificial Sequence

<220>
<223>  primer 6r-bio

<400>  7
atcaggggtg ggtacaaatc ctaattgc                                         28

<210>  8
<211>  28
<212>  DNA
<213>  Artificial Sequence

<220>
<223>  primer 7r-bio

<400>  8
atctggtgca ggaacaaatc ctaattgc                                         28

<210>  9
<211>  28
<212>  DNA
<213>  Artificial Sequence

<220>
<223>  primer 8r-bio

<400>  9
gggaggtggt ggaacaaaag ctaattgc                                         28

<210>  10
<211>  21
<212>  DNA
<213>  Human papillomavirus

<220>
<221>  misc_feature
<222>  (1)..(21)
<223>  consensus region in HPV4


<400>  10
atggctcatt taaatgtaat g                                                21


<210>  11
<211>  21
<212>  DNA
<213>  Human papillomavirus

<220>
<221>  misc_feature
<222>  (1)..(21)
<223>  consensus region in HPV5


<400>  11
ttggcacaga tcaatgcaat g                                                21


<210>  12
<211>  21
<212>  DNA
<213>  Human papillomavirus

<220>
<221>  misc_feature
<222>  (1)..(21)
<223>  consensus region in HPV8


<400>  12
ttagcacaaa tcaatgccat g                                                21


<210>  13
<211>  21
<212>  DNA
<213>  Human papillomavirus

<220>
<221>  misc_feature
<222>  (1)..(21)
<223>  consensus region in HPV9


<400>  13
ttatcccaga taaatgcaat g                                                21

<210> 14
<211> 21
<212> DNA
<213> Human papillomavirus

<220>
<221> misc_feature
<222> (1)..(21)
<223> consensus region in HPV12


<400> 14
ttagcacaga taaatgcaat g                                                21


<210> 15
<211> 21
<212> DNA
<213> Human papillomavirus

<220>
<221> misc_feature
<222> (1)..(21)
<223> consensus region in HPV14


<400> 15
ttagctcaaa ttaatgcaat g                                                21


<210> 16
<211> 21
<212> DNA
<213> Human papillomavirus

<220>
<221> misc_feature
<222> (1)..(21)
<223> consensus region in HPV15


<400> 16
ttaacacaaa ttaatgctat g                                                21


<210> 17
<211> 21
<212> DNA
<213> Human papillomavirus

<220>
<221> misc_feature
<222> (1)..(21)
<223> consensus region in HPV17


<400> 17
ttaactcaaa ttaatgcaat g                                                21

<210>  18
<211>  21
<212>  DNA
<213>  Human papillomavirus

<220>
<221>  misc_feature
<222>  (1)..(21)
<223>  consensus region in HPV19


<400>  18
ctggcacaaa tcaatgcaat g                                                21


<210>  19
<211>  21
<212>  DNA
<213>  Human papillomavirus

<220>
<221>  misc_feature
<222>  (1)..(21)
<223>  consensus region in HPV20


<400>  19
ttagctcaaa ttaatgctat g                                                21


<210>  20
<211>  21
<212>  DNA
<213>  Human papillomavirus

<220>
<221>  misc_feature
<222>  (1)..(21)
<223>  consensus region in HPV21


<400>  20
ttagctcaaa ttaatgcaat g                                                21


<210>  21
<211>  21
<212>  DNA
<213>  Human papillomavirus

<220>
<221>  misc_feature
<222>  (1)..(21)
<223>  consensus region in HPV22


<400>  21
ttaactcaaa ttaatgccat g                                                21


<210>  22

<211>  21
<212>  DNA
<213>  Human papillomavirus

<220>
<221>  misc_feature
<222>  (1)..(21)
<223>  consensus region in HPV23


<400>  22
ttaacacaaa ttaatgccat g                                                21


<210>  23
<211>  21
<212>  DNA
<213>  Human papillomavirus

<220>
<221>  misc_feature
<222>  (1)..(21)
<223>  consensus region in HPV24


<400>  23
ttagcacaga tcaatgctat g                                                21


<210>  24
<211>  21
<212>  DNA
<213>  Human papillomavirus

<220>
<221>  misc_feature
<222>  (1)..(21)
<223>  consensus region in HPV25


<400>  24
ttggctcaga ttaatgcaat g                                                21


<210>  25
<211>  21
<212>  DNA
<213>  Human papillomavirus

<220>
<221>  misc_feature
<222>  (1)..(21)
<223>  consensus region in HPV36


<400>  25
ttggctcaga taaatgctat g                                                21


<210>  26
<211>  21

<212>  DNA
<213>  Human papillomavirus

<220>
<221>  misc_feature
<222>  (1)..(21)
<223>  consensus region in HPV37


<400>  26
ttaactcaga taaatgcaat g                                                21


<210>  27
<211>  21
<212>  DNA
<213>  Human papillomavirus

<220>
<221>  misc_feature
<222>  (1)..(21)
<223>  consensus region in HPV38


<400>  27
ctgacacaga ttaatgctat g                                                21


<210>  28
<211>  21
<212>  DNA
<213>  Human papillomavirus

<220>
<221>  misc_feature
<222>  (1)..(21)
<223>  consensus region in HPV47


<400>  28
ttagcacaaa ttaatgccat g                                                21


<210>  29
<211>  21
<212>  DNA
<213>  Human papillomavirus

<220>
<221>  misc_feature
<222>  (1)..(21)
<223>  consensus region in HPV48


<400>  29
ctggcacatt tacacgcttt g                                                21


<210>  30
<211>  21
<212>  DNA

<213>  Human papillomavirus

<220>
<221>  misc_feature
<222>  (1)..(21)
<223>  consensus region in HPV49


<400>  30
ctggcacaaa tcaatgctat g                                                21


<210>  31
<211>  21
<212>  DNA
<213>  Human papillomavirus

<220>
<221>  misc_feature
<222>  (1)..(21)
<223>  consensus region in HPV50


<400>  31
ttagctcatc ttaatgtaat g                                                21


<210>  32
<211>  21
<212>  DNA
<213>  Human papillomavirus

<220>
<221>  misc_feature
<222>  (1)..(21)
<223>  consensus region in HPV60


<400>  32
ttagctcatt taaatgtaat g                                                21


<210>  33
<211>  21
<212>  DNA
<213>  Human papillomavirus

<220>
<221>  misc_feature
<222>  (1)..(21)
<223>  consensus region in HPV65


<400>  33
atggctcact taaatgtaat g                                                21


<210>  34
<211>  28
<212>  DNA
<213>  Human papillomavirus

<220>
<221> misc_feature
<222> (1)..(28)
<223> consensus region in HPV4

<400> 34
gggaggtggt ggaacaaaat ttaactgc 28

<210> 35
<211> 28
<212> DNA
<213> Human papillomavirus

<220>
<221> misc_feature
<222> (1)..(28)
<223> consensus region in HPV5

<400> 35
atcaggagtg ggaacaaatc ctaactgc 28

<210> 36
<211> 28
<212> DNA
<213> Human papillomavirus

<220>
<221> misc_feature
<222> (1)..(28)
<223> consensus region in HPV8

<400> 36
atcaggggta ggtacaaatc ccagttgc 28

<210> 37
<211> 28
<212> DNA
<213> Human papillomavirus

<220>
<221> misc_feature
<222> (1)..(28)
<223> consensus region in HPV9

<400> 37
ttcaggtgat ggaacaaacc ctaattgc 28

<210> 38
<211> 28
<212> DNA
<213> Human papillomavirus

<220>
<221> misc_feature
<222> (1)..(28)
<223> consensus region in HPV12

<400> 38
gtcaggagtt ggcacgaagc ctaattgc 28

<210> 39
<211> 28
<212> DNA
<213> Human papillomavirus

<220>
<221> misc_feature
<222> (1)..(28)
<223> consensus region in HPV14

<400> 39
gtctggtgca ggtacaaatc ctaattgc 28

<210> 40
<211> 28
<212> DNA
<213> Human papillomavirus

<220>
<221> misc_feature
<222> (1)..(28)
<223> consensus region in HPV15

<400> 40
gtctggtgta ggaacaaacc ctagttgc 28

<210> 41
<211> 28
<212> DNA
<213> Human papillomavirus

<220>
<221> misc_feature
<222> (1)..(28)
<223> consensus region in HPV17

<400> 41
atctggtgta ggcacaaatc ctaactgc 28

<210> 42
<211> 28
<212> DNA
<213> Human papillomavirus

<220>

<221>  misc_feature
<222>  (1)..(28)
<223>  consensus region in HPV19


<400>  42
atcgggtgca ggcacgaaac ctaattgc                                          28


<210>  43
<211>  28
<212>  DNA
<213>  Human papillomavirus

<220>
<221>  misc_feature
<222>  (1)..(28)
<223>  consensus region in HPV20


<400>  43
atccggtgca ggaacgaatc ctaactgc                                          28


<210>  44
<211>  28
<212>  DNA
<213>  Human papillomavirus

<220>
<221>  misc_feature
<222>  (1)..(28)
<223>  consensus region in HPV21


<400>  44
gtctggggca ggaacaaatc ctaactgc                                          28


<210>  45
<211>  28
<212>  DNA
<213>  Human papillomavirus

<220>
<221>  misc_feature
<222>  (1)..(28)
<223>  consensus region in HPV22


<400>  45
gtctggtgta ggtacaaagc ctagttgc                                          28


<210>  46
<211>  28
<212>  DNA
<213>  Human papillomavirus

<220>
<221>  misc_feature

<222>  (1)..(28)
<223>  consensus region in HPV23


<400>  46
atctggtgta ggaacaaacc ctaactgc                                          28


<210>  47
<211>  28
<212>  DNA
<213>  Human papillomavirus

<220>
<221>  misc_feature
<222>  (1)..(28)
<223>  consensus region in HPV24


<400>  47
gtctggtgca ggcacaaatc ctaactgc                                          28


<210>  48
<211>  28
<212>  DNA
<213>  Human papillomavirus

<220>
<221>  misc_feature
<222>  (1)..(28)
<223>  consensus region in HPV25


<400>  48
gtccggtgca ggaacgaatc ctaattgc                                          28


<210>  49
<211>  28
<212>  DNA
<213>  Human papillomavirus

<220>
<221>  misc_feature
<222>  (1)..(28)
<223>  consensus region in HPV36


<400>  49
atctggagta ggtacaaaac ctaactgc                                          28


<210>  50
<211>  28
<212>  DNA
<213>  Human papillomavirus

<220>
<221>  misc_feature
<222>  (1)..(28)

<223>  consensus region in HPV37

<400>  50
atctggagta ggtacaaatc ctaattgc                                          28

<210>  51
<211>  28
<212>  DNA
<213>  Human papillomavirus

<220>
<221>  misc_feature
<222>  (1)..(28)
<223>  consensus region in HPV38

<400>  51
gtctgggtgg ggtacaaagc ctaattgc                                          28

<210>  52
<211>  28
<212>  DNA
<213>  Human papillomavirus

<220>
<221>  misc_feature
<222>  (1)..(28)
<223>  consensus region in HPV47

<400>  52
gtctggagta ggcacaaatc ctaactgc                                          28

<210>  53
<211>  28
<212>  DNA
<213>  Human papillomavirus

<220>
<221>  misc_feature
<222>  (1)..(28)
<223>  consensus region in HPV48

<400>  53
tggagctggt ggaacaaaag ctaattgc                                          28

<210>  54
<211>  28
<212>  DNA
<213>  Human papillomavirus

<220>
<221>  misc_feature
<222>  (1)..(28)
<223>  consensus region in HPV49

<400>  54
atcaggggta ggaacaaatc ccaattgc                                          28


<210>  55
<211>  28
<212>  DNA
<213>  Human papillomavirus

<220>
<221>  misc_feature
<222>  (1)..(28)
<223>  consensus region in HPV50


<400>  55
gggagctggt ggaactaaag ctaattgc                                          28


<210>  56
<211>  28
<212>  DNA
<213>  Human papillomavirus

<220>
<221>  misc_feature
<222>  (1)..(28)
<223>  consensus region in HPV60


<400>  56
tggtggtgga ggtacaaatg aaagttgc                                          28


<210>  57
<211>  28
<212>  DNA
<213>  Human papillomavirus

<220>
<221>  misc_feature
<222>  (1)..(28)
<223>  consensus region in HPV65


<400>  57
aggaggaggg ggcacaaaat ttaattgc                                          28


<210>  58
<211>  30
<212>  DNA
<213>  Artificial Sequence

<220>
<223>  generic detection probe SkinPR 1-DIG

<220>
<221>  misc_feature

<222>  (10)..(10)
<223>  "S" on pos. 10 stands for C or G


<220>
<221>  misc_feature
<222>  (12)..(12)
<223>  "H" on pos. 12 stands for A, T or C


<220>
<221>  misc_feature
<222>  (15)..(15)
<223>  "Y" on pos. 15 stands for C or T


<220>
<221>  misc_feature
<222>  (18)..(18)
<223>  "W" on pos. 18 stands for A or T


<220>
<221>  misc_feature
<222>  (21)..(24)
<223>  "R" on pos. 21 and 24 stand for C or T


<220>
<221>  misc_feature
<222>  (27)..(27)
<223>  "D" on pos. 27 stands for A, T or G


<400>  58
gcaatgaats chaayatwtt rgargadtgg                                        30


<210>  59
<211>  30
<212>  DNA
<213>  Artificial Sequence

<220>
<223>  generic detection probe SkinPR 2-DIG

<220>
<221>  misc_feature
<222>  (9)..(9)
<223>  "Y" on pos. 9 stands for C or T


<220>
<221>  misc_feature
<222>  (12)..(12)
<223>  "W" on pos. 12 stands for A or T


<220>
<221>  misc_feature
<222>  (13)..(13)

<223>  "D" on pos. 13 stands for A, T or G

<220>
<221>  misc_feature
<222>  (14)..(14)
<223>  "V" on pos. 14 stands for A, C or G

<220>
<221>  misc_feature
<222>  (18)..(24)
<223>  "R" on pos. 18 and 24 stand for A or G

<400>  59
gcaatgaayt cwdvtatrtt ggargattgg                                        30

<210>  60
<211>  30
<212>  DNA
<213>  Artificial Sequence

<220>
<223>  generic detection probe SkinPR 3-DIG

<220>
<221>  misc_feature
<222>  (9)..(10)
<223>  "Y" on pos. 9 and 10 stand for C or T

<220>
<221>  misc_feature
<222>  (12)..(16)
<223>  "H" on pos. 12 and 16 stand for A, T or C

<220>
<221>  misc_feature
<222>  (13)..(27)
<223>  "D" on pos. 13, 15 and 27 stand for A, T or G

<220>
<221>  misc_feature
<222>  (18)..(18)
<223>  "W" on pos. 18 stands for A or T

<220>
<221>  misc_feature
<222>  (21)..(24)
<223>  "R" on pos. 21 and 24 stand for A or G

<400>  60
gcaatgaayy chdvdhtwtt rgargadtgg                                        30

<210> 61
<211> 30
<212> DNA
<213> Human papillomavirus

<220>
<221> misc_feature
<222> (1)..(30)
<223> probe-binding region in DNA of cutaneous supergroup B HPV


<400> 61
gtaatgaatc ctaatatttt ggataattgg 30


<210> 62
<211> 30
<212> DNA
<213> Human papillomavirus

<220>
<221> misc_feature
<222> (1)..(30)
<223> probe-binding region in DNA of cutaneous supergroup B HPV


<400> 62
gcaatgaact cttcgttatt ggaggattgg 30


<210> 63
<211> 30
<212> DNA
<213> Human papillomavirus

<220>
<221> misc_feature
<222> (1)..(30)
<223> probe-binding region in DNA of cutaneous supergroup B HPV


<400> 63
gccatgaatt catcactact tgaggaatgg 30


<210> 64
<211> 30
<212> DNA
<213> Human papillomavirus

<220>
<221> misc_feature
<222> (1)..(30)
<223> probe-binding region in DNA of cutaneous supergroup B HPV


<400> 64
gcaatgaact caggtatttt agaggattgg 30

<210> 65
<211> 30
<212> DNA
<213> Human papillomavirus

<220>
<221> misc_feature
<222> (1)..(30)
<223> probe-binding region in DNA of cutaneous supergroup B HPV


<400> 65
gcaatgaact cttctttgct ggaggactgg 30


<210> 66
<211> 30
<212> DNA
<213> Human papillomavirus

<220>
<221> misc_feature
<222> (1)..(30)
<223> probe-binding region in DNA of cutaneous supergroup B HPV


<400> 66
gcaatgaatt ctaatatttt agaggagtgg 30


<210> 67
<211> 30
<212> DNA
<213> Human papillomavirus

<220>
<221> misc_feature
<222> (1)..(30)
<223> probe-binding region in DNA of cutaneous supergroup B HPV


<400> 67
gctatgaatt caggtatttt agaagactgg 30


<210> 68
<211> 30
<212> DNA
<213> Human papillomavirus

<220>
<221> misc_feature
<222> (1)..(30)
<223> probe-binding region in DNA of cutaneous supergroup B HPV


<400> 68
gcaatgaact caggaattct ggaagactgg 30


<210> 69

<211>  30
<212>  DNA
<213>  Human papillomavirus

<220>
<221>  misc_feature
<222>  (1)..(30)
<223>  probe-binding region in DNA of cutaneous supergroup B HPV


<400>  69
gcaatgaatt ctaacatatt ggaagaatgg                                        30


<210>  70
<211>  30
<212>  DNA
<213>  Human papillomavirus

<220>
<221>  misc_feature
<222>  (1)..(30)
<223>  probe-binding region in DNA of cutaneous supergroup B HPV


<400>  70
gctatgaatt caaatatatt agaggagtgg                                        30


<210>  71
<211>  30
<212>  DNA
<213>  Human papillomavirus

<220>
<221>  misc_feature
<222>  (1)..(30)
<223>  probe-binding region in DNA of cutaneous supergroup B HPV


<400>  71
gcaatgaatg caaatatttt agaagaatgg                                        30


<210>  72
<211>  30
<212>  DNA
<213>  Human papillomavirus

<220>
<221>  misc_feature
<222>  (1)..(30)
<223>  probe-binding region in DNA of cutaneous supergroup B HPV


<400>  72
gccatgaatc atggcatttt agaaaattgg                                        30


<210>  73
<211>  30

<212>  DNA
<213>  Human papillomavirus

<220>
<221>  misc_feature
<222>  (1)..(30)
<223>  probe-binding region in DNA of cutaneous supergroup B HPV


<400>  73
gccatgaatt cagatatttt agagaattgg                                        30


<210>  74
<211>  30
<212>  DNA
<213>  Human papillomavirus

<220>
<221>  misc_feature
<222>  (1)..(30)
<223>  probe-binding region in DNA of cutaneous supergroup B HPV


<400>  74
gctatgaatc catctctatt ggaagagtgg                                        30


<210>  75
<211>  29
<212>  DNA
<213>  Human papillomavirus

<220>
<221>  misc_feature
<222>  (1)..(30)
<223>  probe-binding region in DNA of cutaneous supergroup B HPV


<400>  75
gcaatgaatt ccaacatttt agaagatgg                                         29


<210>  76
<211>  30
<212>  DNA
<213>  Human papillomavirus

<220>
<221>  misc_feature
<222>  (1)..(30)
<223>  probe-binding region in DNA of cutaneous supergroup B HPV


<400>  76
gctatgaatt cttctttatt ggaagattgg                                        30


<210>  77
<211>  30
<212>  DNA

<213>  Human papillomavirus

<220>
<221>  misc_feature
<222>  (1)..(30)
<223>  probe-binding region in DNA of cutaneous supergroup B HPV


<400>  77
gcaatgaatt ctggtatatt ggaagagtgg                                        30


<210>  78
<211>  30
<212>  DNA
<213>  Human papillomavirus

<220>
<221>  misc_feature
<222>  (1)..(30)
<223>  probe-binding region in DNA of cutaneous supergroup B HPV


<400>  78
gctatgaatt ctggaatatt agaaaattgg                                        30


<210>  79
<211>  30
<212>  DNA
<213>  Human papillomavirus

<220>
<221>  misc_feature
<222>  (1)..(30)
<223>  probe-binding region in DNA of cutaneous supergroup B HPV


<400>  79
gccatgaatt cgtctctttt agaggaatgg                                        30


<210>  80
<211>  30
<212>  DNA
<213>  Human papillomavirus

<220>
<221>  misc_feature
<222>  (1)..(30)
<223>  probe-binding region in DNA of cutaneous supergroup B HPV


<400>  80
gttatgaatc ccagaatatt agaggagtgg                                        30


<210>  81
<211>  30
<212>  DNA
<213>  Human papillomavirus

<220>
<221>  misc_feature
<222>  (1)..(30)
<223>  probe-binding region in DNA of cutaneous supergroup B HPV

<400>  81
gctatgaatt cttctatatt ggaaaattgg                                        30

<210>  82
<211>  30
<212>  DNA
<213>  Human papillomavirus

<220>
<221>  misc_feature
<222>  (1)..(30)
<223>  probe-binding region in DNA of cutaneous supergroup B HPV

<400>  82
gtaatggacc ccagaatttt ggaaaattgg                                        30

<210>  83
<211>  30
<212>  DNA
<213>  Human papillomavirus

<220>
<221>  misc_feature
<222>  (1)..(30)
<223>  probe-binding region in DNA of cutaneous supergroup B HPV

<400>  83
gtaatggaca aaaatatttt agaggattgg                                        30

<210>  84
<211>  30
<212>  DNA
<213>  Human papillomavirus

<220>
<221>  misc_feature
<222>  (1)..(30)
<223>  probe-binding region in DNA of cutaneous supergroup B HPV

<400>  84
gtaatgaatc ctaatatctt agacaattgg                                        30

<210>  85
<211>  23
<212>  DNA
<213>  Artificial Sequence

<220>
<223>  type-specific detection probe RLBskinHPV4

<400>  85
aatcctaata ttttggataa ttg                                              23


<210>  86
<211>  20
<212>  DNA
<213>  Artificial Sequence

<220>
<223>  type-specific detection probe RLBskinHPV 5

<400>  86
tcttcgttat tggaggattg                                                  20


<210>  87
<211>  20
<212>  DNA
<213>  Artificial Sequence

<220>
<223>  type-specific detection probe RLBskinHPV 8

<400>  87
aattcatcac tacttgagga                                                  20


<210>  88
<211>  20
<212>  DNA
<213>  Artificial Sequence

<220>
<223>  type-specific detection probe RLBskinHPV 9

<400>  88
tcaggtattt tagaggattg                                                  20


<210>  89
<211>  18
<212>  DNA
<213>  Artificial Sequence

<220>
<223>  type-specific detection probe RLBskinHPV 12

<400>  89
ttctttgctg gaggactg                                                    18


<210>  90
<211>  22
<212>  DNA
<213>  Artificial Sequence

<220>

<223>  type-specific detection probe RLBskinHPV 14

<400>  90
attctaatat tttagaggag tg                                                22


<210>  91
<211>  22
<212>  DNA
<213>  Artificial Sequence

<220>
<223>  type-specific detection probe RLBskinHPV 15

<400>  91
aattcaggta ttttagaaga ct                                                22


<210>  92
<211>  20
<212>  DNA
<213>  Artificial Sequence

<220>
<223>  type-specific detection probe RLBskinHPV 17

<400>  92
aactcaggaa ttctggaaga                                                   20


<210>  93
<211>  21
<212>  DNA
<213>  Artificial Sequence

<220>
<223>  type-specific detection probe RLBskinHPV 19

<400>  93
ttctaacata ttggaagaat g                                                 21


<210>  94
<211>  22
<212>  DNA
<213>  Artificial Sequence

<220>
<223>  type-specific detection probe RLBskinHPV 20

<400>  94
attcaaatat attagaggag tg                                                22


<210>  95
<211>  22
<212>  DNA
<213>  Artificial Sequence

<220>
<223>  type-specific detection probe RLBskinHPV 21

<400>  95
atgcaaatat tttagaagaa tg                                                22

<210>  96
<211>  22
<212>  DNA
<213>  Artificial Sequence

<220>
<223>  type-specific detection probe RLBskinHPV 22

<400>  96
aatcatggca ttttagaaaa tt                                                22

<210>  97
<211>  22
<212>  DNA
<213>  Artificial Sequence

<220>
<223>  type-specific detection probe RLBskinHPV 23

<400>  97
attcagatat tttagagaat tg                                                22

<210>  98
<211>  19
<212>  DNA
<213>  Artificial Sequence

<220>
<223>  type-specific detection probe RLBskinHPV 24

<400>  98
catctctatt ggaagagtg                                                    19

<210>  99
<211>  22
<212>  DNA
<213>  Artificial Sequence

<220>
<223>  type-specific detection probe RLBskinHPV 25

<400>  99
aattccaaca ttttagaaga at                                                22

<210>  100
<211>  21
<212>  DNA
<213>  Artificial Sequence

<220>
<223>  type-specific detection probe RLBskinHPV 36

<400>  100
ttcttcttta ttggaagatt g                                                21


<210>  101
<211>  20
<212>  DNA
<213>  Artificial Sequence

<220>
<223>  type-specific detection probe RLBskinHPV 37

<400>  101
ttctggtata ttggaagagt                                                  20


<210>  102
<211>  22
<212>  DNA
<213>  Artificial Sequence

<220>
<223>  type-specific detection probe RLBskinHPV 38

<400>  102
attctggaat attagaaaat tg                                               22


<210>  103
<211>  20
<212>  DNA
<213>  Artificial Sequence

<220>
<223>  type-specific detection probe RLBskinHPV 47

<400>  103
aattcgtctc ttttagagga                                                  20


<210>  104
<211>  19
<212>  DNA
<213>  Artificial Sequence

<220>
<223>  type-specific detection probe RLBskinHPV 48

<400>  104
cccagaatat tagaggagt                                                   19


<210>  105
<211>  22
<212>  DNA
<213>  Artificial Sequence

<220>
<223>  type-specific detection probe RLBskinHPV 49

<400>  105

attcttctat attggaaaat tg                                              22

<210>  106
<211>  20
<212>  DNA
<213>  Artificial Sequence

<220>
<223>  type-specific detection probe RLBskinHPV 50

<400>  106
accccagaat tttggaaaat                                                20

<210>  107
<211>  22
<212>  DNA
<213>  Artificial Sequence

<220>
<223>  type-specific detection probe RLBskinHPV 60

<400>  107
gacaaaaata ttttagagga tt                                              22

<210>  108
<211>  22
<212>  DNA
<213>  Artificial Sequence

<220>
<223>  type-specific detection probe RLBskinHPV 65

<400>  108
aatcctaata tcttagacaa tt                                              22

<210>  109
<211>  72
<212>  DNA
<213>  Human papillomavirus

<220>
<221>  misc_feature
<222>  (1)..(72)
<223>  part of HPV type 4 genome

<400>  109
atggctcatt taaatgtaat gaatcctaat attttggata attggcagtt aaattttgtt      60

ccaccacctc cc                                                          72

## Description

### Field of the invention

The invention relates to a method for detecting and typing of human papillomavirus (HPV). In particular, the invention relates to a method for detecting and typing cutaneous HPVs of supergroup B. Also, the invention relates to primers and probes for use in a method of the invention.

### Background of the invention

The etiologic agents of all warts are a diverse group of viruses known as human papillomaviruses (HPVs) that, in all, constitute a group of more than 100 types of viruses, as identified by variations in DNA sequence. The various HPVs cause a variety of cutaneous and mucosal diseases. Certain types may cause warts, or papillomas, which are benign (noncancerous) tumors. Others have been found to cause invasive carcinoma of the uterine cervix.

Of all HPV types, more than 30 can be passed from one person to another through sexual contact. HPV infection is thereby one of the most common sexually transmitted diseases (STDs). Lesions may appear within several weeks to several months after sexual contact with a person who has HPV, or they may never appear, since HPV infections often do not cause any symptoms. There is currently no medical cure to eliminate a papillomavirus infection, although the lesions and warts themselves can be treated.

Papillomaviruses together with the Polyomaviruses belong to the Papovaviridae, a family of small, non-enveloped tumor viruses with a double stranded DNA genome. Individual isolates are highly host specific. Papillomaviruses specific for human (HPV), bovine (BPV), avian (e.g. FPV), deer (DPV), Cottontail rabbit (CRPV), European elk (EEPV) and numerous other animals have been identified.

Unlike other viruses that are mostly grouped by serotypes, papillomaviruses are classified by genotypes for the same host and are numbered in the order of identification (e.g. HPV1-HPV83 and BPV1-BPV4).

HPVs are broadly classified into low-risk and high-risk types, based on their ability to induce malignant changes in infected cells. Low risk HPV types such as 1, 2, 4, 6, 11, 13 and 32 are primarily associated with benign lesions or common warts while the high risk types, such as 16, 18, 31, 33, 35, 39, 45, 51, 52, 56, 58, 59, 68, and 69 are primarily associated with premalignant and malignant epithelial lesions. These high-risk types of HPV cause growths that are usually flat and nearly invisible, as compared with the warts caused by low-risk types, e.g. HPV-6 and HPV-11.

Papillomaviruses can in general also be classified according to tissue tropism. Although all known human papillomaviruses strictly infect cells of epithelial origin (keratinocytes) and induce 'epithelial tumors' they can be grouped according to their tissue tropism into either mucosal or cutaneous HPV types.

The mucosal types (e.g. HPV6 and HPV16), include those HPVs that infect mucosa of the genital and respiratory tracts. The cutaneous types (e.g. HPV1 and HPV8), include those HPVs that infect generally the external skin, including those that cause cutaneous warts and those that cause skin carcinomas in patients suffering from epidermodysplasia verruciformis (EV; a rare hereditary disease characterized by disseminated flat warts and macular lesions arising during childhood and by a predisposition to HPV-induced skin carcinoma later in life).

The HPV types that have been shown to be associated with EV constitute a group of cutaneous HPVs classified as EV-type (e.g. types 5, 8, 17, 20 and 36). Some of these viruses may cause cancer in genetically predisposed individuals only. Based on their phylogenetic relationship the EV-type HPVs together with the EV-type related HPVs and type 4 HPVs are collectively classified as the supergroup B-type and form the largest group of cutaneous HPV types.

The above is articulated to indicate that, generally, the type of HPV predicts the site of the infection and its clinical course.

Recent data suggest that the supergroup B-type cutaneous human papillomaviruses might be involved in the pathogenesis of non-melanoma skin cancer (NMSC), particularly in immunosuppressed (e.g. renal transplant recipients) patients. In addition, cutaneous HPV DNA, especially of types 5, 36 and 38 can be detected in up to 90% of psoriasis lesions, suggesting an etiological relationship. Studies on cutaneous HPVs are, however, hampered by the fact that these comprise a large and highly heterogeneous virus group for which a plurality of PCR-based detection systems in parallel need to be employed.

The cutaneous HPV detection and typing systems of the prior art have a number of limitations. One disadvantage of the HPV detection and typing systems of the prior art is that only a restricted number of HPV types can be detected in a single assay. Therefore several separate PCR assays need to be performed to cover a broad range of different types (see e.g. Shamanin *et al.,* 1996, J. Natl. Cancer Inst. 88: 802-811; Meijer *et al.,* 2001, Cancer Detect. Prev. 25: 533-547).

Further, the assays of the prior art require relatively large DNA fragments (Shamanin *et al.,* 1996, J. Natl. Cancer Inst. 88: 802.811; Berkhout *et al.,* 1995, J. Clin. Microbiol. 33: 690-695; Forslund *et al.,* 1999, J. Gen. Virol. 80: 2437-2443; Meijer *et al.,* 2001, Cancer Detect. Prev. 25: 533-547). Since both appropriate preservation of material (e.g. formalin-fixation) as well as poor preservation of clinical material may yield degraded DNA, such assays can only suitably be performed on relatively fresh, unfixed material. This hampers their use and prevents standardization of methodologies.

Moreover, the current assay systems that are suitable for detection of cutaneous HPVs have no system for typing coupled thereto. As a result, laborious reamplification, cloning and sequencing of DNA amplification products is required for typing of HPVs (Shamani *et al.,* 1996, J. Natl. Cancer Inst. 88: 802-811; Berkhout *et al.,* 1995, J. Clin. Microbiol. 33: 690-695).

Another problem of some methods for detection of multiple HPV types at respectable sensitivity of the prior art is that their format is a so-called nested-PCR format (Berkhout *et al.,* 1995, J. Clin. Microbiol. 33: 690-695). Such methods are extremely sensitive to contamination, as very small amounts of contaminating template DNA will already yield false positive results. Moreover, such a format is very laborious.

### Summary of the invention

In order to overcome these difficulties the inventors have now found a method for the detection of any of the various cutaneous HPV types that belong to supergroup B. Furthermore, and in an additional aspect of the invention, the inventors have found a method for an efficient typing system for HPV types belonging to supergroup B that is compatible with the method for detection.

A method for the detection of cutaneous supergroup B HPVs according to the present invention comprises the steps of providing a sample suspected of harbouring cutaneous supergroup B HPVs, providing a plurality of pairs of bi-directional primers collectively substantially complementary to DNA of all cutaneous supergroup B HPVs, performing a reaction to amplify DNA derived from the said sample using said plurality of primers; and detecting DNA amplification products from cutaneous supergroup B HPV by hybridising the reaction products of the said DNA amplification reaction to a plurality of generic cutaneous supergroup B HPV probes.

In an additional aspect, the invention provides a method for typing of a cutaneous supergroup B HPV comprising the steps of providing DNA amplification products by amplifying DNA of cutaneous supergroup B HPV between a plurality of pairs of bi-directional primers, and detecting DNA amplification products from one or more supergroup B HPV types by hybridising the said amplification products to at least one cutaneous supergroup B HPV probe that is substantially complementary to the DNA of at least one but not all cutaneous supergroup B HPV types.

In a further aspect, the invention provides a set of bi-directional amplification primers for the amplification of supergroup B HPV-DNA.

In another aspect, the invention provides generic oligonucleotide probes for the generic detection of supergroup B HPV-DNA.

In yet another aspect, the invention provides type-specific oligonucleotide probes for the type-specific or selective detection of individual HPV-types belonging to supergroup B of HPV.

### Description of the drawings

Figure 1 presents suitable pairs of bi-directional primers for the amplification of DNA from cutaneous HPV belonging to supergroup B.
Figure 2 presents the sequences of supergroup B HPV-specific primer-binding regions in DNA of cutaneous HPV belonging to supergroup B (5'- 3'). For clarity reasons and to facilitate alignment of the sequences presented, the complementary strands of these regions are not shown, but are understood to be included.
Figure 3 presents suitable generic detection probes for detection of amplicons derived from cutaneous supergroup B HPV (5'DIG-3'). As illustrated in the text, these probes may also consist of the complementary strands of those shown.
Figure 4 presents the sequences of probe-binding region in DNA of cutaneous supergroup B HPV (5'- 3'). For clarity reasons and to facilitate alignment of the sequences presented, the complementary strands of these regions are not shown, but are understood to be included.
Figure 5 presents type-specific detection probes suitable for reverse line blot (RLB) typing of amplicons derived from cutaneous supergroup B HPV (5'amino-3'). As illustrated in the text, these probes may also consist of the complementary strands of those shown.

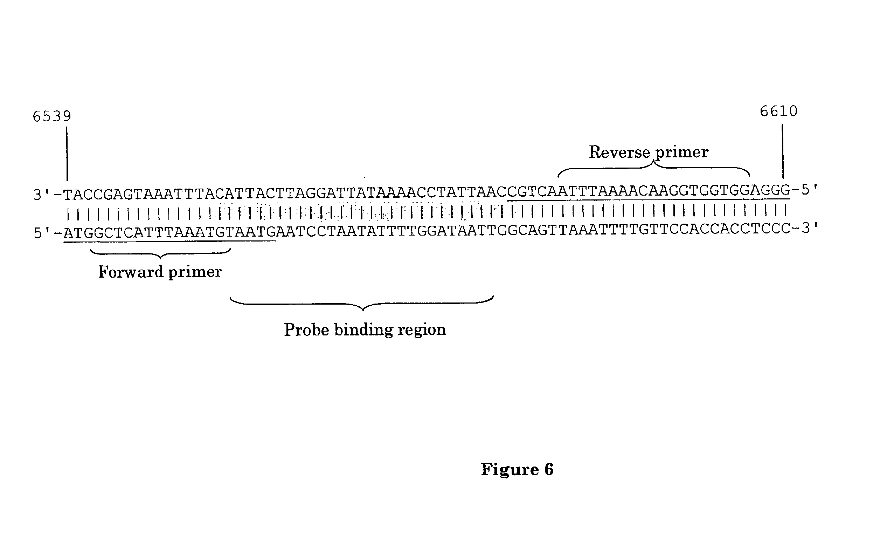

Figure 6 presents the double stranded DNA sequence of HPV type 4 (numbering according to EMBL accession number X70827). Indicated are positions of bi-directional primers (underlined) and probe-binding region (shaded) as defined in Figures 2 and 4, respectively.

### Detailed description of the invention

A "complement" or "complementary sequence" is a sequence of nucleotides which forms a hydrogen-bonded duplex with another sequence of nucleotides according to Watson-Crick base-paring rules. For example, the complementary base sequence for 5'-AAGGCT-3' is 3'-TTCCGA-5'.

"Expression" refers to the transcription of a gene into structural RNA (rRNA, tRNA) or messenger RNA (mRNA) and, if applicable, subsequent translation into a protein.

Polynucleotides are "heterologous" to one another if they do not naturally occur together in the same organism. A polynucleotide is heterologous to an organism if it does not naturally occur in its particular form and arrangement in that organism.

Polynucleotides have "homologous" sequences if the sequence of nucleotides in the two sequences is the same when aligned for maximum correspondence as described herein. Sequence comparison between two or more polynucleotides is generally performed by comparing portions of the two sequences over a comparison window to identify and compare local regions of sequence similarity. The comparison window is generally from about 20 to 200 contiguous nucleotides. The "percentage of sequence homology" for polynucleotides, such as 50, 60, 70, 80, 90, 95, 98, 99 or 100 percent sequence homology may be determined by comparing two optimally aligned sequences over a comparison window, wherein the portion of the polynucleotide sequence in the comparison window may include additions or deletions (i.e. gaps) as compared to the reference sequence (which does not comprise additions or deletions) for optimal alignment of the two sequences. The percentage is calculated by: (a) determining the number of positions at which the identical nucleic acid base occurs in both sequences to yield the number of matched positions; (b) dividing the number of matched positions by the total number of positions in the window of comparison; and (c) multiplying the result by 100 to yield the percentage of sequence homology. Optimal alignment of sequences for comparison may be conducted by computerized implementations of known algorithms, or by inspection. Readily available sequence comparison and multiple sequence alignment algorithms are, respectively, the Basic Local Alignment Search Tool (BLAST) (Altschul, S.F. et al. 1990. J. Mol. Biol. 215:403; Altschul, S.F. et al. 1997. Nucleic Acid Res. 25:3389-3402) and ClustalW programs both available on the internet. Other suitable programs include GAP, BESTFIT and FASTA in the Wisconsin Genetics Software Package (Genetics Computer Group (GCG), Madison, WI, USA).

As used herein, "substantially complementary" means that two nucleic acid sequences have at least about 65%, preferably about 70%, more preferably about 80%, even more preferably 90%, and most preferably about 98%, sequence complementarity to each other. This means that the primers and probes must exhibit sufficient complementarity to their template and target nucleic acid, respectively, to hybridise under stringent conditions. Therefore, the primer sequences as disclosed in this specification need not reflect the exact sequence of the binding region on the template and degenerate primers can be used. A substantially complementary primer sequence is one that has sufficient sequence complementarity to the amplification template to result in primer binding and second-strand synthesis.

The term "hybrid" refers to a double-stranded nucleic acid molecule, or duplex, formed by hydrogen bonding between complementary nucleotides. The terms "hybridise" or "anneal" refer to the process by which single strands of nucleic acid sequences form double-helical segments through hydrogen bonding between complementary nucleotides.

The term "oligonucleotide" refers to a short sequence of nucleotide monomers (usually 6 to 100 nucleotides) joined by phosphorous linkages (e.g., phosphodiester, alkyl and aryl-phosphate, phosphorothioate), or non-phosphorous linkages (e.g., peptide, sulfamate and others). An oligonucleotide may contain modified nucleotides having modified bases (e.g., 5-methyl cytosine) and modified sugar groups (e.g., 2'-O-methyl ribosyl, 2'-O-methoxyethyl ribosyl, 2'-fluoro ribosyl, 2'-amino ribosyl, and the like). Oligonucleotides may be naturally-occurring or synthetic molecules of double-and single-stranded DNA and double- and single-stranded RNA with circular, branched or linear shapes and optionally including domains capable of forming stable secondary structures (e.g., stem-and-loop and loop-stem-loop structures).

The term "primer" as used herein refers to an oligonucleotide which is capable of annealing to the amplification target allowing a DNA polymerase to attach thereby serving as a point of initiation of DNA synthesis when placed under conditions in which synthesis of primer extension product which is complementary to a nucleic acid strand is induced, i.e., in the presence of nucleotides and an agent for polymerization such as DNA polymerase and at a suitable temperature and pH. The (amplification) primer is preferably single stranded for maximum efficiency in amplification. Preferably, the primer is an oligodeoxy ribonucleotide. The primer must be sufficiently long to prime the synthesis of extension products in the presence of the agent for polymerization. The exact lengths of the primers will depend on many factors, including temperature and source of primer. A "pair of bi-directional primers" as used herein refers to one forward and one reverse primer as commonly used in the art of DNA amplification such as in PCR amplification.

The term "probe" refers to a single-stranded oligonucleotide sequence that will recognize and form a hydrogen-bonded duplex with a complementary sequence in a target nucleic acid sequence analyte or its cDNA derivative.

The terms "stringency" or "stringent hybridization conditions" refer to hybridization conditions that affect the stability of hybrids, e.g., temperature, salt concentration, pH, formamide concentration and the like. These conditions are empirically optimised to maximize specific binding and minimize nonspecific binding of primer or probe to its target nucleic acid sequence. The terms as used include reference to conditions under which a probe or primer will hybridise to its target sequence, to a detectably greater degree than other sequences (e.g. at least 2-fold over background). Stringent conditions are sequence dependent and will be different in different circumstances. Longer sequences hybridise specifically at higher temperatures. Generally, stringent conditions are selected to be about 5°C lower than the thermal melting point (Tₘ) for the specific sequence at a defined ionic strength and pH. The Tₘ is the temperature (under defined ionic strength and pH) at which 50% of a complementary target sequence hybridises to a perfectly matched probe or primer. Typically, stringent conditions will be those in which the salt concentration is less than about 1.0 M Na⁺ ion, typically about 0.01 to 1.0 M Na⁺ ion concentration (or other salts) at pH 7.0 to 8.3 and the temperature is at least about 30°C for short probes or primers (e.g. 10 to 50 nucleotides) and at least about 60°C for long probes or primers (e.g. greater than 50 nucleotides). Stringent conditions may also be achieved with the addition of destabilizing agents such as formamide. Exemplary low stringent conditions or "conditions of reduced stringency" include hybridization with a buffer solution of 30% formamide, 1 M NaCl, 1% SDS at 37°C and a wash in 2x SSC at 40°C. Exemplary high stringency conditions include hybridization in 50% formamide, 1 M NaCI, 1% SDS at 37°C, and a wash in 0.1x SSC at 60°C. Hybridization procedures are well known in the art and are described in e.g. Ausubel et al, Current Protocols in Molecular Biology, John Wiley & Sons Inc., 1994.

A method of the present invention permits the detection, identification and diagnosis of human papillomaviruses belonging to supergroup B and human papillomavirus infections in organisms susceptible to such infections that are caused by supergroup B HPV.

### Detection of supergroup B HPVs

A method for detection of supergroup B HPVs according to the present invention may be performed on samples derived from humans, but also on other samples, such as environmental samples, or samples of other localities where HPV may reside, such as samples from other organisms such as insects. Preferably, a sample for use in a method of the invention comprises a human sample, such as a sample of a patient's cells. More preferably, the sample comprises the patient's cells from the site of a potential or suspected infection.

The sample of a patient's cells may comprise a sample of tumorous skin cells (e.g. in the case of warts, veruccas and the like, presumably caused by cutaneous HPV infections). Alternatively, the sample may comprise a sample of tumorous mucosal cells, such as cervical cells in the case of HPV infections of the female urinogenital tract, but also cancerous tissue of lung, larynx, oral cavity, tonsil, oesophagus, penis, vulva, anus, rectum, prostate, ovary and other tissues may be used as a sample in a method of the present invention.

Methods of obtaining and preparing such samples for use in the method of the invention are known to those skilled in the art or will be apparent from the present disclosure. For general methods concerning DNA analysis and manipulations see e.g. Molecular Cloning: A Laboratory Manual, 2nd Ed., Vol. 1-3, eds. Sambrook et al. Cold Spring Harbor Laboratory Press (1989) or Current Protocols in Molecular Biology, eds. Ausubel et al., Greene Publishing and Wiley-Interscience, New York (1987) and periodic updates thereof.

Preferably, a method according to the invention is used for the detection of HPV types 4, 5, 8, 9, 12, 14, 15, 17, 19, 20, 21, 22, 23, 24, 25, 36, 37, 38, 47, 48, 49, 50, 60 and/or 65. All these types are classified as cutaneous supergroup B HPV.

It is an advantageous aspect of the invention that members of the cutaneous HPV supergroup B that are not yet discovered may suitably be detected by using a method of the present invention. Moreover, the method of typing of supergroup B HPVs may be expanded to facilitate for the incorporation of these newly discovered types in a HPV typing system according to the present invention.

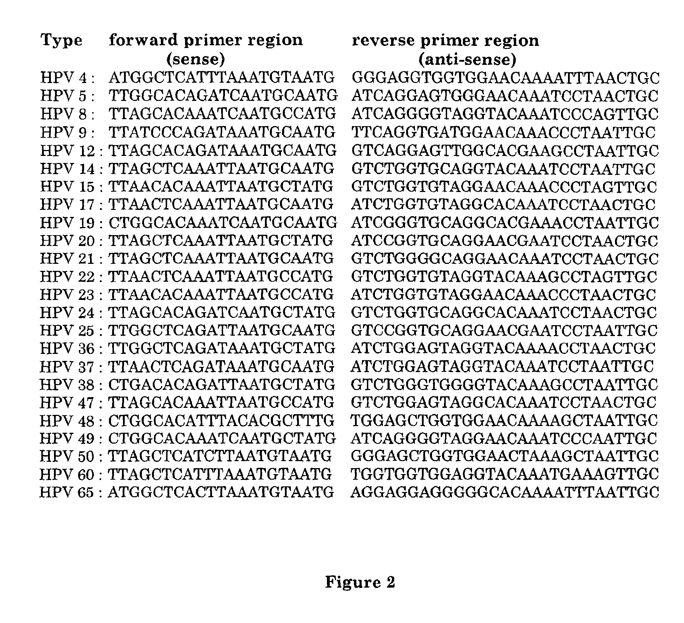

The inventors have now found two discrete regions in the DNA of all cutaneous supergroup B HPV types that are closely spaced together and that appear homologous between the various supergroup B HPV types. These two homologous regions, or consensus regions, are located in the L1 open reading frame (ORF) of the cutaneous supergroup B HPV genome. The DNA sequence of these two consensus regions is presented in Figure 2. The first consensus region comprises a region defined herein as the region extending from about nucleotide position 6539 to about nucleotide position 6559 in a 5'-3' direction on the genome of HPV type 4 (numbering according to EMBL accession number X70827, HPV type 4) and the corresponding region within the genome of other cutaneous supergroup B HPV types. The second consensus region comprises a region defined herein as the region extending from about nucleotide position 6583 until about nucleotide position 6610 in a 5'-3' direction on the HPV 4 genome and the corresponding region within the genome of other cutaneous supergroup B HPV types.

Based on this finding the present invention provides a set of bi-directional primers wherein each individual primer is capable of annealing to one of the two consensus regions of a plurality of cutaneous supergroup B HPV types. A complete set preferably provides a plurality of pairs of bi-directional primers that, when used in concert, are capable of annealing to these two consensus regions in the DNA of any cutaneous supergroup B HPV and of amplifying the defined DNA sequences between and adjacent these two consensus regions, i.e. flanked by these two consensus regions.

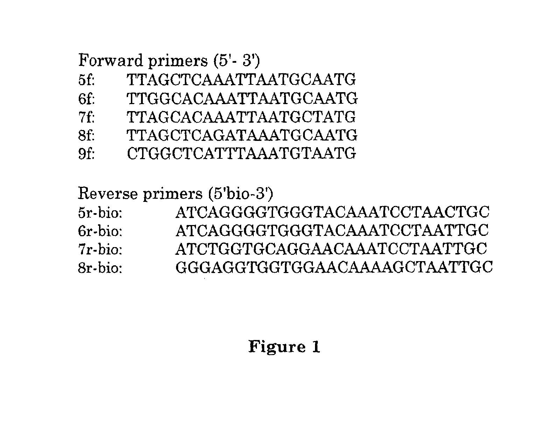

An example of suitable pairs of bi-directional primers to amplify the region between the above mentioned two consensus regions from all cutaneous supergroup B HPVs is presented in Figure 1. From the present disclosure it will be apparent to the person skilled in the art that other sets of primers than those of Figure 1 may be designed and used for amplification of specific sequences flanked by or flanking the two consensus regions in the L1 ORF of the cutaneous supergroup B HPV genome. For instance, it is possible to use somewhat shorter or somewhat longer primers, or primer sequences containing e.g. inositol residues or ambiguous bases or even primers that contain one or more mismatches when compared to the sequences of the primers of Figure 1. In general, sequences that exhibit at least 65%, more preferably at least 80% homology with the oligonucleotide sequences of Figure 1 are considered suitable for use in a method of the present invention.

Methods of the invention can in principle be performed by using any nucleic acid amplification method, such as the Polymerase Chain Reaction (PCR; Mullis 1987, U.S. Pat. No. 4,683,195, 4,683,202, en 4,800,159) or by using amplification reactions such as Ligase Chain Reaction (LCR; Barany 1991, Proc. Natl. Acad. Sci. USA 88:189-193; EP Appl. No., 320,308), Self-Sustained Sequence Replication (3SR; Guatelli *et al.,* 1990, Proc. Natl. Acad. Sci. USA 87:1874-1878), Strand Displacement Amplification (SDA; U.S. Pat. Nos. 5,270,184, en 5,455,166), Transcriptional Amplification System (TAS; Kwoh *et al.,* Proc. Natl. Acad. Sci. USA 86:1173-1177), Q-Beta Replicase (Lizardi *et al.,* 1988, Bio/Technology 6:1197), Rolling Circle Amplification (RCA; U.S. Pat. No. 5,871,921), Nucleic Acid Sequence Based Amplification (NASBA), Cleavase Fragment Length Polymorphism (U.S. Pat. No. 5,719,028), Isothermal and Chimeric Primer-initiated Amplification of Nucleic Acid (ICAN), Ramification-extension Amplification Method (RAM; U.S. Pat. Nos. 5,719,028 and 5,942,391) or other suitable methods for amplification of DNA.

A preferred embodiment according to the present invention uses a PCR method comprising the primer-initiated amplification of supergroup B HPV-DNA with a set of 5 overlapping forward and 4 overlapping reverse primers that together match sequences within the L1 open reading frame of all known HPV types belonging to supergroup B.

Since the two consensus regions in the L1 ORF as defined herein are closely spaced together, a preferred set of bi-directional amplification primers amplifies a short fragment (i.e. about 72 base pairs) of supergroup B HPV-DNA. This provides for the possibility to amplify DNA sequences from template DNA that is of poor quality, such as DNA that is degraded to a certain extent, which allows for the use of formalin fixed specimens as a sample material, as well as of other less well preserved materials for use in a method of the present invention.

In order to amplify the DNA of HPV types with a small number of mismatches with one or more of the amplification primers, an amplification reaction may be performed under conditions of reduced stringency (e.g. a PCR amplification using an annealing temperature of 38°C, or the presence of 3.5 mM MgCl₂). The person skilled in the art will be able to select conditions of suitable stringency.

Primer sequences and corresponding sequences of supergroup B HPV-specific DNA amplification primers are shown in Figure 1.

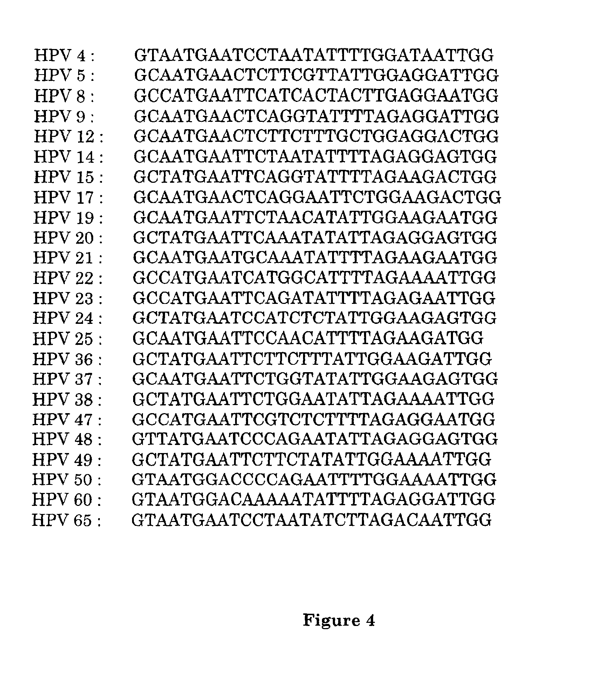

Another important aspect of the sequence region flanked by the two consensus regions in the L1 ORF is that the DNA sequence thereof comprises common motifs for various HPVs belonging to supergroup B, as can be seen in Figure 4.

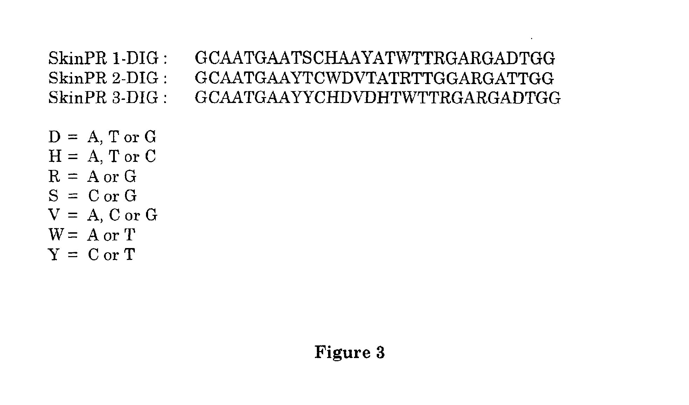

Therefore, by amplifying the DNA sequence region between the two consensus regions in the L1 ORF that are specific for supergroup B HPVs, all supergroup B HPVs may be detected by using a set of generic cutaneous supergroup B HPV detection probes that essentially will hybridise to the amplicons of those HPVs that contain the common motif. When used in concert, such generic detection probes will bind all supergroup B HPVs amplification products. The inventors have found that a very advantageous system employs the hybridization to a cocktail of 3 overlapping generic probes provided with wobbles to detect all possible HPV types amplified by the cutaneous supergroup B HPV primers. An example of a set of suitable generic detection probes that binds to the region between the above mentioned two consensus regions of amplicons of all supergroup B HPVs is presented in Figure 3.

From the present disclosure it will be apparent to the person skilled in the art that other sets of generic detection probes than those of Figure 3 may be designed and used for detection of the DNA sequence flanked by the two consensus regions in the L1 ORF of all cutaneous supergroup B HPVs. For instance, it is possible to use somewhat shorter or somewhat longer probes, or probe sequences containing ambiguous bases or wobbles that are directed to a somewhat different cluster of HPV types, or an otherwise different set of probes than those of Figure 3, such as probes that contain one or more mismatches to their target sequence. In general, sequences that exhibit at least 65%, more preferably at least 80% homology with the oligonucleotide sequences of Figure 3, are considered suitable for use in a method of the present invention for the generic detection of amplicons of all supergroup B HPVs.

Also the complementary sequences of those listed in Figure 3 may suitably be used as generic detection probes in a method of the invention, provided that the complementary strand is amplified in the amplification reaction employed, such as when e.g. PCR is used for amplification.

The detection of the amplification products can in principle be accomplished by any suitable method known in the art. The detection fragments may be directly stained or labelled with radioactive labels, antibodies, luminescent dyes, fluorescent dyes, or enzyme reagents. Direct DNA stains include for example intercalating dyes such as acridine orange, ethidium bromide, ethidium monoazide or Hoechst dyes.

Alternatively, the DNA fragments may be detected by incorporation of labelled dNTP bases into the synthesized DNA fragments. Detection labels which may be associated with nucleotide bases include e.g. fluorescein, cyanine dye or BrdUrd.

The present invention preferably involves the detection of the reaction products obtained by the above described DNA amplification reaction on cutaneous supergroup B HPV DNA by hybridising the reaction products to one or more generic cutaneous HPV supergroup B probes.

When using a probe-based detection system, a suitable detection procedures for use in the present invention may for example comprise an enzyme immunoassay (EIA) format (Jacobs *et al.*, 1997, J. Clin. Microbiol. 35, 791-795). For performing a detection by manner of the EIA procedure, either the forward or the reverse primer used in the amplification reaction may comprise a capturing group, such as a biotin group for immobilization of supergroup B HPV-DNA PCR amplicons on e.g. a streptavidin coated microtiter plate wells for subsequent EIA detection of supergroup B HPV-amplicons (see below). The skilled person will understand that other groups for immobilization of supergroup B HPV-DNA PCR amplicons in an EIA format may be employed.

### Typing of supergroup B HPVs

Another important aspect of the DNA sequence region flanked by the two consensus regions in the L1 ORF of the cutaneous supergroup B HPV is that the DNA sequence thereof is also unique to each HPV type belonging to supergroup B, as can be seen in Figure 4.

Therefore, and in another aspect of the invention, the various HPV types belonging to supergroup B may be detected by using a plurality of type-specific cutaneous supergroup B HPV detection probes that, individually, bind only to the amplicon of one specific type of HPV belonging to supergroup B. Thus, by amplifying the DNA sequence region between the two consensus regions as defined herein, followed by probing for the unique sequences of each individual HPV-type, a method is provided for typing of the various supergroup B HPVs.

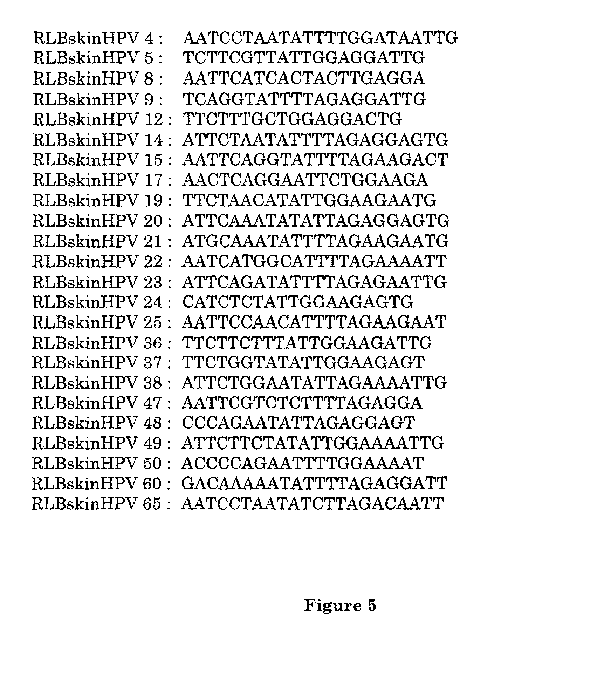

Probes useful for the type-specific detection of the region in the L1 ORF from DNA of supergroup B-type HPVs as disclosed herein are exemplified in Figure 5. These type-specific probes preferably bind only to at least a part of the DNA sequence region as amplified between the two consensus regions of a single HPV-type belonging to supergroup B. Those of skill in the art can prepare similar probes for type-specific detection based on the nucleotide sequence of the L1 ORF from the DNA of supergroup B HPVs without undue experimentation as set out herein. Also the complementary sequences of those listed in Figure 5 may suitably be used as type-specific detection probes in a method of the invention, provided that such a complementary strand is amplified in the amplification reaction employed.

Probes used in a method of typing according to the invention bind selectively to the amplicon of a certain HPV type or types, but not - or only weakly under hybridization conditions of very low stringency - to the amplicon of other HPV types. Accordingly, in one embodiment, the invention can be used to determine the type or types of HPV infecting a patient. This is very significant, as progression to malignant disease (and hence clinical prognosis) is heavily dependent on HPV type. Accordingly, in a typing aspect, the invention provides a method of determining the type(s) of HPV infection in a patient.

In a method of typing of supergroup B HPVs according to the invention, suitable probes are for instance those that hybridize to the amplicons of only a subset of HPV types. Such probes may for instance be the generic probes of Figure 3. In such an embodiment, for example, the ability to limit the infecting HPV type(s) to a particular subset (or exclude such a subset) may be sufficient.

In a method of typing of supergroup B HPVs according to the invention, the subset of amplicons to which a typing probe binds may consist of a single HPV type amplicon, or may consist of a plurality of amplicons of different types, such that binding or non-binding (as appropriate) of the type-specific probe to the amplicons generated from the sample will allow an investigator to make certain deductions about the identity of the HPV type(s) infecting the patient. Probes which bind to desired HPV types, but not to undesired HPV types, may be generated by methods known to the skilled person.

The present invention now provides a particular region of the amplicon to which probes may bind with considerable specificity. Although homologous regions may exist in all HPV amplicons generated, the nucleic acid sequence of this particular region is unique for HPVs of different type.

If a similar typing needs to be performed for supergroup B HPVs which are not (yet) included or discovered, clearly, the nucleic acid sequence of the amplicon of such HPV types will not necessarily be complementary to that of any of the probes herein provided. However, with the benefit of the present disclosure a suitable hybridization region can readily be identified in other amplicons by those skilled in the art by use of conventional alignment and sequence comparison computer programs.

In a preferred embodiment of a method for typing of supergroup B HPVs of the present invention a set of 24 different probes (Figure 5), or the complementary sequences thereof, is used that hybridise to the type-specific sequences in the amplicons derivable from the 24 different cutaneous supergroup B HPVs as shown in Figure 4.

Suitable detection procedures for use in the typing aspect of present invention may for example comprise immobilization of the amplicons and probing the DNA sequences thereof by e.g. southern blotting. Other formats may comprise an EIA format as described above. To facilitate the detection of binding, the type-specific supergroup B HPV probes may comprise a label moiety such as a fluorophore, a chromophore, an enzyme or a radio-label, so as to facilitate monitoring of binding of the probes to the reaction product of the amplification reaction. Such labels are well-known to those skilled in the art and include, for example, fluorescein isothiocyanate (FITC), β-galactosidase, horseradish peroxidase, streptavidin, biotin, digoxigenin, ³⁵S or ¹²⁵I. Other examples will be apparent to those skilled in the art.

Although other methods may be employed, a method of typing according to the invention is preferably performed by a so called reverse line blot (RLB) assay, such as recently developed for e.g. mucosotropic HPVs (van den Brule *et al.,* 2002, J. Clin. Microbiol. 40, 779-787). For this purpose RLB probes are preferably synthesized with a 5'amino group for subsequent immobilization on e.g. carboxyl-coated nylon membranes. The advantage of an RLB format is the ease of the system and its speed, thus allowing for high throughput sample processing.

A method of typing of supergroup B HPVs according to the invention may be performed on similar sample material as that used for the detection of supergroup B HPVs described above. Alternatively, the typing of the supergroup B HPV according to a method of the invention may be performed directly on amplicons or DNA amplification reaction products obtained by using the DNA amplification reaction as described for the detection of supergroup B HPV herein above.

As the difference between the two aspects of a method of the invention is in the application of different detection probes, a method of the invention may in one aspect comprise the amplification of suspected supergroup B HPV DNA, followed by the detection of amplicons of any HPV (i.e. of an infection with supergroup B HPV) by using a generic detection probe cocktail that, in concert, hybridises to all supergroup B HPV amplicons generated. In another aspect, a method of the invention may comprise the amplification of supergroup B HPV DNA, followed by the typing for 24 HPVs in, for example a reverse line blot assay. Thus, detection and typing according to methods of the invention may be performed in parallel.

### Primers and probes

In a further aspect, the invention provides a set of bi-directional amplification primers for the amplification of supergroup B HPV-DNA for use in a method of the invention. These primers are preferably oligonucleotide primers with a sequence length of about 8 to about 35, preferably of about 15 to about 30, more preferably of about 21 to about 28 nucleotides. In a most preferred embodiment the forward primer comprises about 21 nucleotides, while the reverse primer comprises about 28 nucleotides. Preferably one of the primers is labelled with a group to facilitate the immobilization of one of strands of the double stranded supergroup B HPV amplicons and in en even more preferred embodiment the reverse primer is labelled with biotin.

The nucleotide sequence of the bi-directional primers of the present invention should be designed to allow the formation of a stable hybrid between the primer and the target DNA at the position of one of the two consensus regions in the L1 ORF of the supergroup B HPV genome as defined herein. Thereto, the nucleotide sequence of the forward primer is preferably substantially complementary to the anti-sense strand of the L1 ORF in the genome of one or more supergroup B HPVs in the region defined as forward primer region in Figure 2, while the nucleotide sequence of the reverse primer is preferably substantially complementary to the sense strand of the L1 ORF in the genome of one or more supergroup B HPVs in the region defined as reverse primer region in Figure 2. Primers that are consistent with this criterion are for example those displayed in Figure 1. When used in concert, the primers of Figure 1 will amplify DNA of a corresponding region in the genome of all supergroup B HPVs.

The primers herein are selected to be "substantially" complementary (i.e. at least 65%, more preferably at least 80% perfectly complementary) to the consensus regions present on the different strands of each specific sequence to be amplified. It is possible to use somewhat shorter or somewhat longer primers, or primer sequences containing e.g. inositol residues or ambiguous bases or even primers that contain one or more mismatches when compared to the sequences of the primers of Figure 1. In general, sequences that exhibit at least 65%, more preferably at least 80% homology with the oligonucleotide sequences of Figure 1, are considered suitable for use in a method of the present invention. Sequence mismatches are also not critical when using low stringency hybridization conditions. In the present invention, the primers are preferably substantially complementary to the sequence of at least one of the two consensus regions in the L1 ORF of supergroup B HPVs.

In another aspect, the invention provides oligonucleotide probes for the generic detection of supergroup B HPV-DNA. The detection probes herein are selected to be "substantially" complementary to one of the strands of the double stranded DNA amplicons generated by an amplification reaction of the invention. Preferably the probes are substantially complementary to the immobilizable, e.g. biotin labelled, antisense strands of the amplicons generated from the L1 ORF of the supergroup B HPVs.

The amplicons comprise regions characterized by common motifs as well as unique sequences between the various supergroup B HPVs. The generic probes for detection of these amplicons are designed to be sufficiently complementary to hybridise with either strand of the amplicons generated from one or more supergroup B HPV types. The generic detection probe sequence need not reflect the exact sequence of either strand of the amplicons, nor does the generic detection probe sequence need to reflect the exact sequence of all supergroup B HPVs. The present invention now provides in Figure 3 a set of probes with ambiguous bases (wobbles) to facilitate their binding to at least a subset of all possible amplicons generated from the various supergroup B HPVs. This minimizes the number of generic probes required for detection of all supergroup B HPVs.

It is allowable for generic detection probes of the present invention to contain one or more mismatches to their target sequence. In general, sequences that exhibit at least 65%, more preferably at least 80% homology with the oligonucleotide sequences of Figure 3, are considered suitable for use in a method of the present invention for the generic detection of amplicons of all supergroup B HPVs. Therefore, when referring to the primers or probes of Figures 1 and 3, it is intended that sequences exhibiting at least 65%, more preferably at least 80% homology with those of Figures 1 and 3 are included.

Also the complementary sequences of those listed in Figure 3 may be used as generic detection probes in a method of the invention, provided that the complementary strand is amplified in the amplification reaction employed.

Probes which bind to some supergroup B HPV types, but not to other supergroup B HPV types, may be generated by methods known to the skilled person.

To facilitate detection the generic supergroup B HPV probes may comprise a label moiety such as a fluorophore, a chromophore, an enzyme or a radio-label, so as to facilitate monitoring of binding of the probes to the reaction product of the amplification reaction. Such labels are well-known to those skilled in the art and include, for example, fluorescein, cyanine dye, β-galactosidase, horseradish peroxidase, streptavidin, biotin, ³⁵S or ¹²⁵I. Other examples will be apparent to those skilled in the art. A preferred label for a generic supergroup B HPV probe used in a method of the present invention is a digoxigenin (DIG) label.

These generic detection probes are preferably oligonucleotide probes with a sequence length of about 8 to about 70, preferably of about 15 to about 40, more preferably of about 25 to about 35 nucleotides. In a most preferred embodiment the generic detection probes comprise about 30 nucleotides.

In yet another aspect, the invention provides oligonucleotide probes for the type-specific or selective detection of individual HPV-types or subsets of types belonging to supergroup B of HPV. In a preferred embodiment one probe is provided for each HPV type, such probes therefore being type-specific. The present invention discloses a set of 24 suitable type-specific probe sequences for the type-specific detection of the specific supergroup B HPVs mentioned in Figure 5. Although some mismatches would be allowable it is preferred that the typing probes are perfectly complementary to their respective target sequences. As outlined above, the complementary sequences of those presented in Figure 5 may also be used as type specific probes in methods of the present invention, provided that the amplification reaction allows for the amplification of the complementary DNA fragments. Therefore, when referring to the probes of figures 3 and 5, it is intended that complementary sequences thereof are included.

Type-specific or selective detection probes can be used in a variety of typing procedures. Suitable procedures comprise those whereby the probe-target is immobilized, but also procedures whereby the probes are immobilized may be used. As examples of the latter, both biochip-type of formats, but also reverse line blot formats, such as RLB or Line Probe Assays may be used.
Depending on the substrate for immobilization and the surface chemistry selected, the skilled person is able to chose a suitable group with which the oligonucleotide probe is labelled for substrate immobilization. In a preferred embodiment this group is an amino-group introduced at the 5'-end of the probe.

The type-specific detection probes are preferably oligonucleotide probes with a sequence length of about 8 to about 30, preferably of about 15 to about 25, more preferably of about 19 to about 23 nucleotides. As with the design of the generic detection probes, the skilled person is aware that the annealing temperature of individual probes within a set of probes that is used in one hybridization reaction may suitable be adjusted by providing balance between G+C content and length of the probe.

In one embodiment the present invention also provides a kit comprising primers and probes for performing a method of the invention. A preferred embodiment of such a kit comprises both the amplification primers, the generic detection probes and the type-specific probes as disclosed in Figures 1, 3, and 5, respectively.

The following non-limiting examples illustrate several embodiments of the present invention.

### Example 1.

Amplification of HPVs of supergroup B, EIA detection and RLB typing.

### Methodology

### HPV test panel.

For the evaluation of the sensitivity and specificity of the RLB typing method, plasmid clones of the following HPV types were used as test panel: HPV 4, 5, 8, 9, 14, 17, 19, 21, 22, 23, 25, 36, 38, 49, and 50.

### Clinical specimens.

For the evaluation of HPV detection and typing on clinical specimens, formalin-fixed, paraffin embedded tissues of skin samples were used. A series of 5 µm sections were cut using the so- called sandwich method (Walboomers et al., 1999, J. Pathol. 189:12-19). Briefly, outer sections were haematoxylineosin stained for histological analyses, whereas inner sections (in total approximately 1 cm² of tissue) were placed into a tube and used for PCR purposes. 250 µl lysis mix (10 mM Tris-HCl (pH 7.5) , 0.45% Tween 20 and 500 µg proteinase K (Roche, Mannheim, Germany) ) was added and incubation was performed overnight at 37°C. The sample was boiled and centrifuged 1 min in a standard table top centrifuge (10,000 rpm). After cooling 10 µl was used for PCR.

### Cutaneous HPV supergroup B-PCR.

Isolated HPV plasmid DNA or pre-treated samples were subjected to PCR using a mixture of 5 forward primers (i.e. 5f, 6f, 7f, 8f, 9f; Figure 1) and 3 biotinylated reverse primers (i.e. 5r-bio, 6r-bio, 7r-bio, and 8r-bio; Figure 1). The PCR reaction mixtures of 50 µl contained 1.5 mM MgCl₂, 200 µM of each dNTP, 12.5 pmol of each primer, 1 unit AmpliTaq Gold DNA polymerase (Perkin-Elmer, Foster City, CA), and various concentrations of HPV plasmid DNA or 10 µl of crude sample extract, in Taq Gold buffer (Perkin-Elmer, Foster City, CA). 40 PCR cycles were performed using a PE 9700 thermocycler (Perkin-Elmer, Foster City, CA). Each PCR reaction was initiated by a preheating step for 10 minutes at 94°C followed by 40 cycles consisting of a denaturation step (20 seconds at 94°C), a primer annealing step (30 seconds at 38°C), and an elongation step (80 seconds at 71°C). Ramping times were as described before (van den Brule et al., 2002, J. Clin. Microbiol. 40: 779-787). The final elongation step was extended for 4 minutes.

### Enzyme-Immunoassay of PCR products.

The EIA was performed using streptavidine-coated microtitre plates (Roche, Mannheim, Germany) and a read-out after overnight incubation as previously described (Jacobs et al., 1997, J. Clin. Microbiol. 35:791-795), except that a hybridisation temperature of 55°C was used. A mixture of 3 DIG-labelled generic oligoprobes (ie. SkinPR 1-DIG, SkinPR 2-DIG, and SkinPR3-DIG; Figure 3) was used for hybridisation.

### HPV reverse line blotting (RLB) of PCR products.

RLB analyses were performed using a system that previously has been developed for mucosotropic HPV types (van den Brule et al., 2002, J. Clin. Microbiol. 40: 779-787). The system is based on the use of a miniblotter for spotting in parallel up to 42 different oligoprobes containing a 5'-amino group on a carboxyl-coated nylon membrane. Subsequently, up to 42 PCR products can be pipetted into the parallel channels of the miniblotter in such way that the channels are perpendicular to the rows of oligoprobes deposited previously. In this format hybridisation is carried out, followed by incubation of the membrane with anti-biotin conjugate and Enhanced Chemiluminescense (ECL) detection.

The HPV-specific 5' amino-linked oligonucleotides (Isogen, Maarssen, The Netherlands) selected for RLB are shown in Figure 5. Selection was performed in such a way that Tₘ was (approximately) 55°C and the length was approximately 20 bp using primer/probe selection software (Oligo). The oligoprobes were covalently bound to negatively charged membranes (Biodyne C, Pall BioSupport, East Hills, NY). Briefly, membranes were activated by using 16 % (wt/vol) EDAC (1-ethyl-3-(3-dimethylaminopropyl)carbodiimide; Sigma, St. Louis, MO) and placed on a miniblotter system (MN 45, Immunetics, Cambridge, MA). The oligonucleotides were applied in parallel lines and after a 1 minute incubation the channels were aspirated and the membrane inactivated using 100 mM NaOH followed by washing in 2x SSPE (0.18 M NaCl, 10mM NaH₂PO₄ and 1 mM EDTA (pH 7.7); Life Technologies, Rockville, MD) supplemented with 0.1% SDS (BDH, Dorset, England).

For hybridisation, 10 µl of biotinylated PCR product was diluted in 150 µl of 2x SSPE/0.1%SDS, heat-denatured (96°C) and rapidly cooled on ice. The PCR products were pipetted into the parallel channels perpendicular to the rows of oligoprobes. Hybridisation was performed at 42°C for 1 hour, followed by two washings in 2x SSPE/0.5%SDS at 51°C. Subsequently, membranes were incubated with streptavidin-peroxidase conjugate for 45 to 60 min at 42° C (1:4000 times diluted in 2x SSPE/0.5% SDS; Roche, Mannheim, Germany), washed twice in 2x SSPE/0.5%SDS for 10 min at 42°C, and rinsed twice in 2x SSPE for 5 min at room temperature. Detection was done, by using chemoluminiscent ECL detection liquid (Amersham, Buckinghamshire, England) followed by an exposure to a film (Hyperfilm, Amersham, Buckinghamshire, England) for 1 to 10 min. Films were subsequently developed. For repeated use, the membranes were stripped by washing two times in 1% SDS at 80°C for 30 min followed by an incubation in 20 mM EDTA (pH 8) at room temperature for 15 min. Membranes were sealed in plastic and stored at 4°C until further use.

### Results

The amplification of HPVs of supergroup B and subsequent EIA detection of PCR products derived from 1 ng of cloned HPVs 4, 5, 8, 9, 14, 17, 19, 21, 22, 23, 25, 36, 38, 49, and 50 revealed clear EIA signals in all cases, whereas no cross-reactivity was observed with human DNA. This indicates a high specificity. Using dilution lines of cloned HPVs 5, 8, 21, and 50 mixed with human placental DNA the cutaneous HPV supergroup B -PCR EIA also performed very well in terms of sensitivity; the analytical sensitivity ranged from 10 fg (HPV 5,and 21) to 100 fg (HPV 8 and 50). Also RLB typing revealed specific signals without cross-reactivity and the sensitivity was equal to if not higher than that of the EIA.

### Example 2.

### Analysis of formalin-fixed clinical specimens

The amplification of cutaneous supergroup B HPV DNA was tested using the materials and methods described in Example 1 except that DNA was used that was isolated from formalin-fixed and paraffin-embedded tissue samples from 87 cutaneous specimens that showed good DNA quality (i.e. positive samples in a beta-globin PCR). These samples included 59 samples of renal transplant recipients, comprising 21 cases of squamous cell carcinoma (SCC), 23 cases of basal cell carcinoma (BCC) and 15 cases of actinic keratosis (AK). Another 29 samples were obtained from immunocompetent patients, comprising 13 cases of SCC, 12 cases of BCC, and 4 cases of AK.

EIA detection with generic supergroup B probes revealed positivity in 62% of the cases: 76% of specimens from renal transplant recipients and 34% of specimens of immunocompetent patients. These included 15 out of 21 cases of SCC (71 %), 15 out of 23 cases of BCC (65%), and 14 out of 15 cases of AK (93%) in samples obtained from renal transplant recipients and 4 out of 13 cases of see (31 %), 2 out of 12 cases of BCC (17%) and 4 out of 4 cases of AK (100 %) in samples obtained from immunocompetent patients.

Typing by RLB of the SCC samples of both renal transplant recipients and immunocompetent patients revealed in between 1 and 7 different types of HPV per sample. All together, the following 19 HPV types were detected in one or more samples: HPV 4, 5, 8, 9, 14, 15, 17, 19, 20, 21, 23, 24, 25, 36, 37, 38, 47, 49, 50. These data are in agreement with data obtained from frozen specimens published in literature using other PCR systems (Shamani *et al.,* 1996, J. Natl. Cancer Inst. 88: 802-811; Berkhout *et al.,* J. Clin. Microbiol. 33: 690-695; Forslund *et al.,* J. Gen. Virol. 80: 2437-2443; Harwood *et al.,* J. Med. Virol. 61: 289-297; Meijer *et al.,* Cancer Detect. Prev. 25: 533-547).

## Claims

1. Method for the detection of cutaneous supergroup B HPVs comprising the steps of:
(a) providing a sample suspected of harbouring cutaneous supergroup B HPVs;
(b) providing a plurality of pairs of bi-directional primers collectively substantially complementary to DNA of all cutaneous supergroup B HPVs;
(c) performing a reaction to amplify DNA derived from the said sample using said plurality of primers; and
(d) detecting DNA amplification products from cutaneous supergroup B HPV from said sample.

2. Method according to claim 1, wherein step (d) is carried out by hybridising the reaction products of the said DNA amplification reaction to a plurality of generic cutaneous supergroup B HPV probes.

3. Method according to claim 1 or 2, wherein said plurality of pairs of bi-directional primers comprises primers that are collectively substantially complementary to a first consensus region in the DNA of all cutaneous supergroup B HPVs, and which plurality further comprises primers that are collectively substantially complementary to a second consensus region in the DNA of all cutaneous supergroup B HPVs.

4. Method according to claim 3, wherein said first and second consensus regions are in the L1 ORF of cutaneous supergroup B HPVs.

5. Method according to claim 4, wherein said first and second consensus regions are substantially as defined in Figure 2.

6. Method according to claim 5, wherein said plurality of pairs of primers comprises the primers of Figure 1.

7. Method according to claim 6, wherein one of each pair of primers of said plurality of pairs of bi-directional primers comprise a biotin label.

8. Method according to any one of the previous claims, wherein said reaction to amplify DNA is performed under conditions of reduced stringency.

9. Method according to any one of the claims 2-8, wherein said plurality of supergroup B HPV probes is substantially complementary to the nucleic acid sequence of the said DNA amplification products from all supergroup B HPVs.

10. Method according to any one of the claims 2-9, wherein said supergroup B HPV probes comprise the probes of Figure 3.

11. Method according to claim 10, wherein said probes comprise a DIG label.

12. Method for typing of cutaneous supergroup B HPVs comprising the steps of:
(a) providing DNA amplification products by amplifying DNA of cutaneous supergroup B HPV using a plurality of pairs of bi-directional primers; and
(b) detecting DNA amplification products from one or more supergroup B HPV types by hybridising the said amplification products to at least one cutaneous supergroup B HPV probe that , is substantially complementary to the DNA of at least one but not all cutaneous supergroup B HPV types.

13. Method according to claim 12, wherein said plurality of pairs of bi-directional primers comprises primers that are collectively substantially complementary to a first consensus region in the DNA of all cutaneous supergroup B HPVs, and which plurality further comprises primers that are collectively substantially complementary to a second consensus region in the DNA of all cutaneous supergroup B HPVs.

14. Method according to claim 13, wherein said first and second consensus regions are in the L1 ORF of cutaneous supergroup B HPVs.

15. Method according to claim 14, wherein said first and second consensus regions are substantially as defined in Figure 2.

16. Method according to claim 15, wherein said plurality of pairs of bi-directional primers comprises the primers of Figure 1.

17. Method according to claim 16, wherein one of each pair of primers of said plurality of pairs of bi-directional primers comprise a biotin label.

18. Method according to any one of claims 12-17, wherein said at least one probe is substantially complementary to the DNA of exactly one type of supergroup B HPV.

19. Method according to any one of the claims 12-18, wherein said at least one probe is selected from the probes of Figure 5.

20. Method according to any one of the claims 12-19, wherein the detection comprises the use of a reverse line blot.

21. Bi-directional primers for use in a method according to any one of the previous claims, which primers are collectively substantially complementary to a first and a second consensus region in the L1 ORF of all supergroup B HPVs.

22. Bi-directional primers of Figure 1.

23. Generic detection probes for the detection of cutaneous supergroup B HPVs, which probes are collectively substantially complementary to a region in the L1 ORF of all supergroup B HPVs between nucleotide positions 6539 and 6610 of HPV 4 and corresponding region of the other cutaneous supergroup B HPV types.

24. Generic detection probes of Figure 3.

25. Detection probes for the detection of cutaneous supergroup B HPV types, which probes are substantially complementary to a region in the L1 ORF of at least one but not all cutaneous supergroup B HPV types between nucleotide positions 6539 and 6610 of HPV 4 and corresponding region of the other cutaneous supergroup B HPV types.

26. Type-specific detection probes for the detection of cutaneous supergroup B HPV types, which probes are substantially complementary to a region in the L1 ORF of exactly one type of cutaneous supergroup B HPV type between nucleotide positions 6539 and 6610 of HPV 4 and corresponding region of the other cutaneous supergroup B HPV types.

27. Type-specific detection probes of Figure 5.
